# EUROPEAN PATENT SPECIFICATION

(11) **EP 4 209 204 B1**
(45) Date of publication and mention of the grant of the patent: **11.09.2024**
(21) Application number: 21871008.5
(22) Date of filing: 27.07.2021
(51) Int. Cl.: A61H 1/02, A61B 5/22, A61H 21/00

(54) **INTELLIGENT MOUTH OPENING REHABILITATION APPARATUS**
INTELLIGENTE REHABILITATIONSVORRICHTUNG FÜR MUNDÖFFNUNG
APPAREIL INTELLIGENT DE RÉÉDUCATION DE L'OUVERTURE DE LA BOUCHE

(30) Priority: 22.09.2020 CN 202011002311
(43) Date of publication of application: 12.07.2023
(73) Proprietor: SHANGHAI NINTH PEOPLE'S HOSPITAL, SHANGHAI JIAOTONG UNIVERSITY SCHOOL OF MEDICINE, Huangpu District Shanghai 200011 (CN)
(72) Inventor: LIU, Jiannan, Shanghai 200011 (CN); ZHAI, Guangtao, Shanghai 200011 (CN); ZHANG, Chenping, Shanghai 200011 (CN); ZHANG, Zhiyuan, Shanghai 200011 (CN)
(74) Representative: Canzler & Bergmeier Patentanwälte Partnerschaft mbB
(86) International application number: PCT/CN2021/108569
(87) International publication number: WO 2022/062630

(56) References cited:
- WO-A1-2015/187949
- CN-A- 110 559 154
- CN-A- 110 559 154
- CN-A- 112 107 457
- CN-U- 208 130 350
- CN-U- 210 009 296
- CN-U- 211 095 769
- CN-U- 211 244 400
- US-A- 4 883 046
- US-A1- 2014 114 146
- US-A1- 2020 113 768

## Description

### Technical Field

The present disclosure relates to the field of oral rehabilitation medical instruments, and in particular, to an intelligent mouth opening rehabilitation device.

CN 110 559 154 A discloses an intelligent mouth opening trainer and a matched intelligent diagnosis and treatment system, including a control module, a timing module, a power module, an input power-supply module, a motion output module and an occlusion plate. The main body of the control module is a single-chip microcomputer WiFi processing transmission chip based on an ESP8266 chip. The timing module is mounted on a circuit board. The main body of the power module is a motor and is directly controlled by the control module. The main body of the input power-supply module is a battery. The motion output module comprises a power transmission lever, one end of which is connected with the motor through a screw and the other side of which is provided with an upper occlusion plate A lower occlusion plate is arranged below the upper occlusion plate and one side of the screw is provided with a spring.

CN 211 244 400 U describes a solution where the head end of an upper connecting rod of a temporal-mandibular joint movement device is connected with an upper mouthpiece sheet, the tail end of the upper connecting rod is connected with a driving wheel, and the tail end of the lower connecting rod is connected with a lower mouthpiece sheet. In addition the tail end of the lower connecting rod is connected to the bottom of a wheel casing.

US 2020/113768 A1 discloses an oral rehabilitation device for rehabilitating the oral cavity of a user that includes a device body, a first activating member, an upper-jaw member, a lower-jaw member, and a processing unit. The first activating member is disposed on an end of the device body. The upper-jaw member and the lower-jaw member are connected to the same end of the device body and are for contacting an upper jaw and lower jaw of the user, respectively. The processing unit is coupled to the first activating member and is used to control the first activating member to drive one of the upper-jaw member and the lower jaw member to open or close relative to the other of the upper-jaw member and the lower-jaw member, such that the upper jaw and the lower jaw can be rehabilitated. The present invention further discloses a medical treatment system to which the oral rehabilitation device is linked.

An involuntary oscillator device is disclosed by US 4 883 046 A having an intraoral-extraoral appliance mounted onto the upper and lower arches. A linear stepper motor forcibly imparts oscillating motion to the appliance. During one-half cycle of oscillation, the lower arch is forced to move away from the upper arch, and during the other one-half cycle the lower arch is forced to move toward the upper arch. A programmable digital computer and an electronic network drive the motor and adjusts the parameters of the oscillating motion.

### Background

A limited mouth opening is one of the most common complications in patients with head and neck cancer after comprehensive treatment. This not only affects a quality of life of patients and even threatens their lives, but also brings a serious social and economic burden. So it is very urgent for oral and maxillofacial surgeons to solve a problem that patients have difficulty in opening their mouth after being treated.

Currently, the main principles of treating the limited mouth opening are to control the progress of the limited mouth opening and to restore functions. However, several approaches to the treatment of mouth opening difficulties have been evaluated by scholars, and current clinical evidences suggest that a maximum mouth opening angle of patients with a mouth opening training intervention or using a mouth opening rehabilitation device is better than that of patients without assisted exercise.

At present, some mouth opening rehabilitation devices are existed on the market. For example, a DynBite temporomandibular joint dynamic stretching rehabilitation device is a rehabilitation product developed by Suzhou MicroPort Ric MedTech Ltd. This rehabilitation device is used to treat patients with the limited mandibular opening caused by muscle injury, surgery, tumor radiation therapy, temporomandibular joint dysfunction, osteoarthritis, post-infection or trauma. The rehabilitation device can relieve patients' difficulty in mouth opening and help them obtain a larger mouth opening angle, thereby restoring normal functions of the temporomandibular joint.

However, most mouth opening rehabilitation devices are still in traditional manual operation modes such as manual measurement, manual recording, etc. So it is very urgent to provide an intelligent mouth-opening rehabilitation device that automatically opens a training angle, automatically obtains a degree of force, automatically assesses pain, automatically records training data and so on.

### Summary

In view of the above-mentioned shortcomings of the prior art, an object of the present disclosure is to provide an intelligent mouth opening rehabilitation device, to solve at least one problem in the prior art.

To achieve the above object and other objects, the present disclosure provides an intelligent mouth opening rehabilitation device. The intelligent mouth opening rehabilitation device includes: a body, an end of the body being provided with an upper bite pad and a lower bite pad; a pressure sensor, provided in the upper bite pad and/or the lower bite pad, for detecting pressure data on the upper bite pad and/or the lower bite pad; a stepping motor, for driving the upper bite pad and/or the lower bite pad to swing longitudinally; and a processor, for controlling the stepping motor to operate or stop according to the pressure data and/or a preset training instruction, thereby driving the upper bite pad and/or the lower bite pad to swing longitudinally or to stop.

According to the invention, the processor controls the stepping motor to operate or stop according to the pressure data, including: the processor compares the pressure data with a preset pressure threshold in real time; if the pressure data are lower than the preset pressure threshold, the processor controls the stepping motor to operate forward, to enable the upper bite pad to swing up a distance and/or the lower bite pad to swing down a distance, then returns to the first operation; and if the pressure data reaches the preset pressure threshold, the processor controls the stepping motor to stop operating, to enable the upper bite pad and/or the lower bite pad to stop swinging.

According to the invention, the processor controls the stepping motor to operate or stop according to the pressure data, including: detecting the pressure data in real time to determine whether the pressure data reach a second pressure threshold, where the second pressure threshold is greater than the preset pressure threshold; and if the pressure data reach the second pressure threshold, the processor controls the stepping motor to operate reversely, thereby enabling the upper bite pad to swing down a distance and/or the lower bite pad to swing up a distance, then returns to the first operation.

In an embodiment, the preset training instruction includes: a preset training duration, and a preset opening angle/ a preset opening distance between the upper bite pad and the lower bite pad.

In an embodiment, an angle sensor is provided in the upper bite pad and/or the lower bite pad and used to collect and characterize an opening angle between the upper bite pad and the lower bite pad; or an infrared rangefinder is provided in the upper bite pad and/or the lower bite pad and used to collect and characterize an opening distance between the upper bite pad and the lower bite pad.

In an embodiment, the processor controls the stepping motor to operate or stop according to the preset opening angle or the preset opening distance in the preset training instruction, including: the processor controls the stepping motor to operate forward according to the received preset opening angle or preset opening distance, to enable the upper bite pad to swing up a distance and/or the lower bite pad to swing down a distance; and when the angle sensor detects that the opening angle between the upper bite pad and the lower bite pad reaches the preset opening angle, or when the infrared rangefinder detects that the opening distance between the upper bite pad and the lower bite pad reaches the preset opening distance, the processor controls the stepping motor to stop operating.

In an embodiment, the processor controls the stepping motor to operate or stop according to the preset training duration in the preset training instruction, including: when the stepping motor starts and enters a stop state, a countdown is carried out based on the preset training duration; and when the timer detects that the training duration has been reached, a speaker emits a prompt tone, and the processor controls the stepping motor to operate reversely, so that the upper bite pad swings down to its starting position and/or the lower bite pad swings up to its starting position.

In an embodiment, the intelligent mouth opening rehabilitation device further includes: a light emitting diode (LED) touch screen, located on one side of the body, used to display real-time pressure data, a training duration, training times, and an opening angle/an opening distance, and/or, used to manually set preset training times and the preset opening angle or the preset opening distance; and a communicator, communicating with an external device, used to send the real-time pressure data, the training duration, the training times, and the opening angle/the opening distance to the external device, and/or, used to receive the preset training times and the preset opening angle or the preset opening distance from the external device.

In an embodiment, a communication mode of the communicator includes a wired communication mode and/or a wireless communication mode; the wired communication mode includes one or a combination of USB1.0/2.0/3.x, Micro USB, Mini USB, serial interface and parallel interface; the wireless communication mode includes one or a combination of 2G/3G/4G/5G, Bluetooth, Infrared, NB-IoT, Rola, Zigbee, MavLink, WIFI, NFC, GPRS, GSM and Ethernet.

In an embodiment, the upper bite pad and the lower bite pad are both provided with a corresponding stepping motor respectively; or, one of the upper bite pad and the lower bite pad is provided with a corresponding stepping motor and the other of the upper bite pad and the lower bite pad is fixed.

As described above, the present disclosure provides an intelligent mouth opening rehabilitation device. The intelligent mouth opening rehabilitation device includes: a body, an end of the body being provided with an upper bite pad and a lower bite pad; a pressure sensor, provided in the upper bite pad and/or the lower bite pad, for detecting pressure data on the upper bite pad and/or the lower bite pad; a stepping motor, for driving the upper bite pad and/or the lower bite pad to swing longitudinally; and a processor, for controlling the stepping motor to operate or stop according to the pressure data and/or a preset training instruction, thereby driving the upper bite pad and/or the lower bite pad to swing longitudinally or to stop.

The following beneficial effects are achieved:

Compared with traditional mouth opening rehabilitation devices that require manual measurement of the opening angle or opening distance per use and require the need to manually control the training duration, the intelligent mouth opening rehabilitation device of the present disclosure controls automatically the upper bite pad and/or the lower bite pad to swing longitudinally, so that the mouth opening training is more accurate, more scientific and more controllable. Therefore, a rehabilitation effect may be greatly improved by using the intelligent mouth opening rehabilitation device in the present disclosure.

### Brief Description of the Drawings

FIG. 1 is a side structural diagram of an intelligent mouth opening rehabilitation device according to an embodiment of the present disclosure.
FIG. 2 is an overall structural diagram of an intelligent mouth opening rehabilitation device according to an embodiment of the present disclosure.

### Detailed Description of the Preferred Embodiments

The embodiments of the present disclosure will be described in detail below with reference to the accompanying drawings, so that those skilled in the art to which the present disclosure pertains can easily implement. The present disclosure can be embodied in many different forms, and is not limited to the embodiments described herein.

In order to clearly describe the present application, components irrelevant to the description are omitted, and the same or similar components throughout the specification are given the same reference numerals.

Throughout the specification, when a component is said to be "connected" to another component, this includes not only the case of "direct connection", but also the case of "indirect connection" with other elements interposed there between. In addition, when a component is said to "include" a certain constituent element, unless there is particularly no description to the contrary, it does not exclude other constituent elements, but means that other constituent elements may also be included.

When an element is said to be "on" another element, it can be directly on the other element, but it can also be accompanied by other elements in between. When an element is referred to as being "directly on" another element, it is not accompanied by the other element in between.

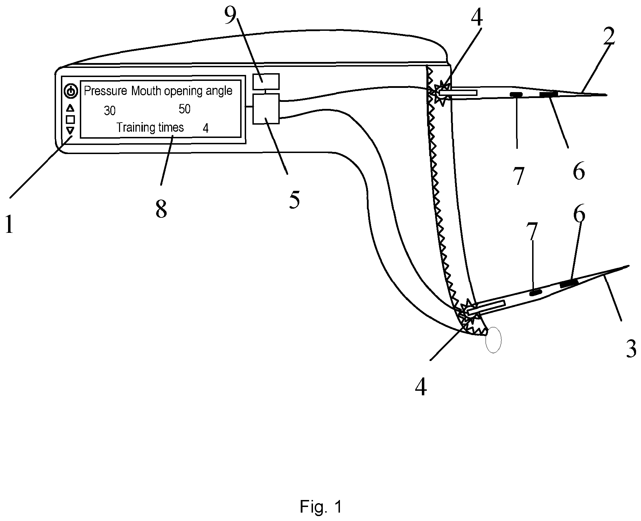

FIG. 1 is a side structural diagram of an intelligent mouth opening rehabilitation device according to an embodiment of the present disclosure. As shown in FIG. 1, the intelligent mouth opening rehabilitation device includes a body 1. An end of the body 1 is provided with an upper bite pad 2 and a lower bite pad 3.

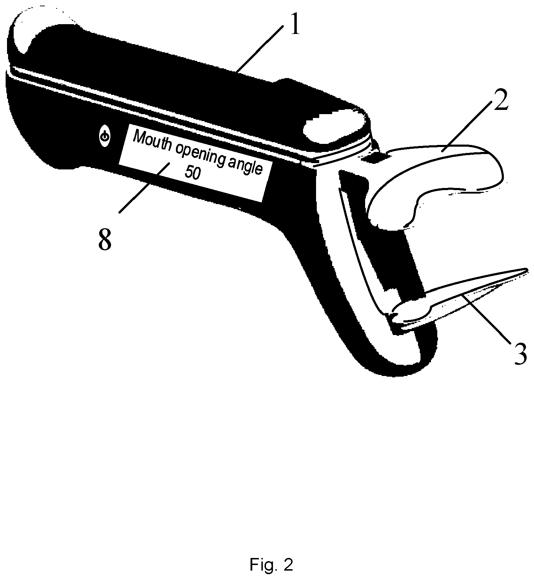

In one or more embodiments, the body 1 is L-shaped or pistol-shaped, and the body 1 includes a transverse part and a longitudinal part. Where the transverse part of the body 1 includes a hand holding part and a light-emitting diode (LED) display part. The longitudinal part of the body 1 corresponds to a bite assembly placed in a user's mouth for a mouth opening training, and is also a teeth supporting part that enters the interior of the user's mouth. In the present disclosure, the above structure of the body 1 fully considers the design principle of ergonomics, the hand holding part is round and full as a whole, the partially sunken human-machine curved surface guides a grasping position of a palm, and a rear end of the hand holding part is designed with an anti-slip surface. FIG. 2 is an overall structural diagram of an intelligent mouth opening rehabilitation device according to an embodiment of the present disclosure.

In an embodiment, the body 1 includes a pressure sensor 6 and a stepping motor 4. The pressure sensor 6 is provided in the upper bite pad 2 and/or the lower bite pad 3, and is for detecting pressure data on the upper bite pad 2 and/or the lower bite pad 3. The stepping motor 4 is for driving the upper bite pad 2 and/or the lower bite pad 3 to swing longitudinally.

In one or more embodiments, a manner in which the stepping motor 4 drives the upper bite pad 2 and/or the lower bite pad 3 to achieve a longitudinal swing includes various common manners. In an embodiment, as shown in FIG. 1, the stepping motor 4 drives a gear to rotate, thereby driving the upper bite pad 2 and/or the lower bite pad 3 to move. In another embodiment, the stepping motor 4 drives a lever, thereby driving the upper bite pad 2 and/or the lower bite pad 3 to swing. Manners of driving the upper bite pad 2 and/or the lower bite pad 3 are not limited in the present disclosure.

In an embodiment, the pressure sensor 6 is only provided in the upper bite pad 2, the stepping motor 4 is set correspondingly for the upper bite pad 2, the lower bite pad 3 is fixed (that is, the lower bite pad 3 may not move or swing), and the stepping motor 4 corresponding to the lower bite pad 3 is not provided. The above structure enables the processor 5 to only control the upper bite pad 2 to swing longitudinally or to stop. In another embodiment, the pressure sensor 6 is only provided in the lower bite pad 3, the stepping motor 4 is set correspondingly for the lower bite pad 3, the upper bite pad 2 is fixed (that is, the upper bite pad 2 may not move or swing), and the stepping motor 4 corresponding to the upper bite pad 2 is not provided. The above structure enables the processor 5 to only control the lower bite pad 3 to swing longitudinally or to stop. In other embodiments, both the upper bite pad 2 and the lower bite pad 3 are provided with the pressure sensors 6 respectively, and the upper bite pad 2 and the lower bite pad 3 are provided with the stepping motors 4 respectively. The above structure enables the processor 5 to simultaneously control the upper bite pad 2 and the lower bite pad 3 to swing longitudinally or to stop, or control the upper bite pad 2 or the lower bite pad 3 to swing longitudinally or to stop.

In addition, existing mouth opening rehabilitation devices do not have a pressure detection device, so during the mouth opening training, patients may only perceive the presence of pain or an opening angle by themselves. However, the pain degree described by patients is not accurate enough for doctors to judge the rehabilitation effect or to formulate the mouth opening training. In addition, most mouth-opening rehabilitation devices have simple mechanical structures without space or foundation to add the pressure sensor 6. In the present disclosure, the pressure sensor 6 detects pressure data on the upper bite pad 2 and/or the lower bite pad 3, and pressure data are displayed on a LED touch screen 8. During the mouth opening training, the doctor may assess the pain degree of the patient at any time according to pressure data displayed on the LED touch screen 8, and then adjust the opening degree of the training and evaluate the degree of rehabilitation. Therefore, an effect of the mouth opening training may be easily measured by the doctors.

The body 1 includes the processor 5. The processor 5 controls the stepping motor 4 to move or stop according to the pressure data and/or a preset training instruction, thereby driving the upper bite pad 2 or the lower bite pad 3 to swing longitudinally or to stop.

In an embodiment, the processor 5 controls the stepping motor 4 to move or stop according to the pressure data, including the following operations:

Operation A, the processor compares the pressure data with a preset pressure threshold in real time.

Optionally, the preset pressure threshold may be a preset pressure threshold for a specific patient obtained by testing the patient's pain performance in combination with the physician's observations when the physician instructs the patient on the use of the mouth opening rehabilitation device in an early stage.

Operation B, if the pressure data are lower than the preset pressure threshold, the processor 5 controls the stepping motor 4 to operate forward, to enable the upper bite pad 2 to swing up a distance and/or the lower bite pad 3 to swing down a distance, then, returning to Operation A.

It should be noted that the distance mentioned here is preferably a driving distance generated by one or more pulse signals of the stepping motor 4. In practice, the distance is less than 10 mm.

Operation C, if the pressure data are not lower than the preset pressure threshold, the processor 5 controls the stepping motor 4 to stop operating, to enable the upper bite pad 2 and/or the lower bite pad 3 to stop swinging. That is, the stepping motor 4 stops operating, then the upper bite pad 2 and/or the lower bite pad 3 are fixed in the current swing state.

When the upper bite pad 2 and/or the lower bite pad 3 swings to be in contact with the teeth of the patient, the pressure data detected by the pressure sensor 6 on the upper bite pad 2 and/or the lower bite pad 3 are increased gradually. So it can be determined that the upper bite pad 2 and/or the lower bite pad 3 has come into contact with the teeth of the patient according to an increasing pressure data, i.e., essentially at the patient's maximum angle of mouth opening. Therefore, the stepping motor 4 stops operating, so that the upper bite pad 2 and/or the lower bite pad 3 are fixed in the current swing state. The rehabilitation training for the patient's mouth opening is realized finally.

For example, when the intelligent mouth opening rehabilitation device is not started, the opening angle between the upper bite pad 2 and the lower bite pad 3 is zero. After the intelligent mouth opening rehabilitation device is sterilized, it may be put into the patient's mouth, then the intelligent mouth opening rehabilitation device is activated. If the training instruction is not set or received from the external device, the intelligent mouth opening rehabilitation device operates by controlling the stepping motor 4 to operate or to stop according to a default pressure data. The pressure sensor 6 provided in the upper bite pad 2 and/or the lower bite pad 3 detects the pressure data in real time. If the pressure data are lower than the preset pressure threshold, the stepping motor 4 operates forward, to enable the upper bite pad 2 to swing up a distance and/or the lower bite pad 3 to swing down a distance. That is, the opening angle between the upper bite pad 2 and the lower bite pad 3 is increased gradually. When the opening angle reaches the maximum angle that the patient's mouth may open, the resistance on the upper bite pad 2 and the lower bite pad 3 will increase continuously, so the pressure data detected by the pressure sensor 6 are also increased continuously. If the pressure data reach the preset pressure threshold, the stepping motor 4 stops operating. So far, one mouth opening training is finished based on the patient's current opening angle.

When the patient wants to interrupt the mouth opening training, or to reduce the opening angle due to increasing soreness of the oral-jaw muscles. In order to make this intelligent mouth opening rehabilitation device more humanized, the processor 5 controlling the stepping motor 4 to operate or stop according to the pressure data further includes:

Operation A, the pressure sensor detects the pressure data in real time to determine whether the pressure data reach a second pressure threshold. Where the second pressure threshold is greater than the preset pressure threshold.

Operation B, if the pressure data reach the second pressure threshold, the processor 5 controls the stepping motor 4 to operate reversely, thereby enabling the upper bite pad 2 to swing down a distance and/or the lower bite pad 3 to swing up a distance, and returning to Operation A.

When the patient wants to interrupt the mouth opening training, the patient may exert a pressure on the upper bite pad 2 and the lower bite pad 3 through the upper and lower jaws of the mouth to reduce the opening angle. Or when the patient wants to reduce the opening angle due to increasing soreness of the oral-jaw muscles after a long period of fixation, the patient may exert a pressure on the upper bite pad 2 and the lower bite pad 3 through the upper and lower jaws of the mouth to reduce the opening angle.

In an embodiment, the training instruction includes: a preset training duration, and/or a preset opening angle or a preset opening distance between the upper bite pad 2 and the lower bite pad 3.

It should be noted that the training instruction may be a reasonable and scientific training setting or planning set by doctors or patients according to the difficulty of mouth opening. For example, doctors or patients may increase the opening angle gradually or increase the training duration gradually according to patients' rehabilitation. So the training instruction may be used not only for a single training, but also for a quantitative, timed, long-term training program in the rehabilitation cycles. Because the intelligent mouth opening rehabilitation device and the remote device may communicate with each other through the training data (e.g., training data include pressure peaks, training times, the opening angle/the opening distance for each or more trainings), and doctors can obtain training data received in the background and provide real-time feedback and advice on the patient's training at home with this intelligent mouth opening rehabilitation device. For example, the intelligent mouth opening rehabilitation device sends training data to an application (APP), and the APP sends feedback (e.g., diet advice, training level advice) automatically to patients according training data.

Compared with traditional mouth opening rehabilitation devices that require manual measurement of the opening angle or opening distance per use and require the need to manually control the training duration, the intelligent mouth opening rehabilitation device in the present disclosure controls automatically the upper bite pad and/or the lower bite pad to swing longitudinally, so that the mouth opening training is more accurate, more scientific and more controllable. Therefore, the rehabilitation effect can be greatly improved by using the intelligent mouth opening rehabilitation device in the present disclosure.

Referring to FIG. 1, a reference numeral 7 represents an angle sensor, or an infrared rangefinder. For ease of understanding, both the angle sensor and the infrared rangefinder described in the present disclosure are represented by the reference numeral 7.

In an embodiment, the angle sensor 7 is provided in the upper bite pad 2 and/or the lower bite pad 3, and used to detect the opening angle between the upper bite pad 2 and the lower bite pad 3. In another embodiment, the infrared rangefinder 7 is provided in the upper bite pad 2 and/or the lower bite pad 3, and used to detect the opening distance between the upper bite pad 2 and the lower bite pad 3.

The opening angle or the opening distance indicates the size of the patient's mouth opening, which indirectly reflects the difficulty of the patient's mouth opening. Based on this, the opening angle or the opening distance may be gradually increased to help patients restore their mouth opening scientifically and reasonably.

In an embodiment, the processor 5 controls the stepping motor 4 to operate or stop according to the preset opening angle or the preset opening distance in the preset training instruction, which includes:

Operation A, the processor 5 controls the stepping motor 4 to operate forward according to the preset opening angle or the preset opening distance, to enable the upper bite pad 2 to swing up a distance and/or the lower bite pad 3 to swing down a distance.

Operation B, when the angle sensor 7 detects that the opening angle between the upper bite pad 2 and the lower bite pad 3 reaches the preset opening angle, or when the infrared rangefinder 7 detects that the opening distance between the upper bite pad 2 and the lower bite pad 3 reaches the preset opening distance, the processor 5 controls the stepping motor 4 to stop operating.

In an embodiment, the processor 5 controls the stepping motor 4 to operate or stop according to the preset training duration in the preset training instruction, which includes:

Operation A, when the stepping motor starts and enters a stop state, a countdown is carried out based on the preset training duration;

Operation B, when the timer detects that the training duration has been reached, a speaker emits a prompt tone, and the processor 5 controls the stepping motor 4 to operate reversely, so that the upper bite pad 2 swings down to its starting position and/or the lower bite pad 3 swings up to its starting position.

In an embodiment, the processor 5 controls the stepping motor 4 to operate or stop according to one of the preset opening angle/the preset opening distance or the preset training duration in the preset training instruction. In another embodiment, the processor 5 also controls the stepping motor 4 to operate or stop according to a combination of the preset opening angle or preset opening distance with the preset training duration in the preset training instruction.

For example, the processor 5 first controls the stepping motor 4 to enable the upper bite pad 2 and/or the lower bite pad 3 to swing to a corresponding opening angle or a corresponding opening distance according to the preset opening angle or the preset opening distance, and then controls the stepping motor 4 to fix or pause the swing angle of the upper bite pad 2 and/or the lower bite pad 3 according to the preset training duration. Based on above processes, the mouth opening training for patients is realized.

In an embodiment, the intelligent mouth opening rehabilitation device includes a light emitting diode (LED) touch screen 8 and a communicator 9. The light emitting diode touch screen 8 is located on one side of the body 1, and used to display real-time pressure data, a training duration, training times, and an opening angle/an opening distance; and/or, used to manually set preset training times and the preset opening angle or the preset opening distance. The communicator 9 communicates with an external device and used to send the real-time pressure data, the training duration, the training times, and the opening angle/the opening distance to the external device; and/or, used to receive preset training times and the preset opening angle/the preset opening distance from the external device.

The training instruction may be manually set through the LED touch screen 8, or input from the external device through the communicator 9.

The communicator 9 communicates with the external device to transmit pressure data. In an embodiment, the external device may be smart phones, smart watches, training applications, hospital data systems, training data platforms and the like of the doctors or patients. So doctors or systems can assess the patient's training and rehabilitation progress, and provide advice, according to training data recorded remotely by the external device. Or, training data may be synchronized to a mobile device at any time. The user may read the data and modify the information of the intelligent mouth opening rehabilitation device through an applet or APP, and upgrade a system of the intelligent mouth opening rehabilitation device through Over-the-Air Technology (OTA) to optimize functions and fix bugs.

For example, if a patient has severe pain during the process of using the intelligent mouth opening rehabilitation device, the pain information may be immediately sent to the doctor through the APP and the patient is also informed to stop training. For example, The APP will record the degree of mouth opening before and after each training, provide dietary advice, and conduct regular assessments of the degree of mouth opening training. The doctor will conduct real-time monitoring through a background and provide timely guidance. The LED touch screen 8 is used for real-time self-control of the patient during the process of the mouth opening training.

In an embodiment, a communication mode of the communicator 9 includes a wired communication mode and/or a wireless communication mode. The wired communication mode includes one or a combination of USB1.0/2.0/3.x, Micro USB, Mini USB, serial interface and parallel interface (e.g., RS485, Standard Parallel Port (SPP)). The wireless communication mode includes one or a combination of 2G/3G/4G/5G, Bluetooth, Infrared, Narrow Band Internet of Things (NB-IoT), Long Range Radio (Rola), Zigbee, Micro Air Vehicle Link (MavLink), WIFI, Near Field Communication (NFC), General packet radio service (GPRS), Global System for Mobile Communications (GSM) and Ethernet.

Optionally, the processor 5, the communicator 9, and the LED touch screen 8 may be integrated together. In addition, all or part of the processor 5, the communicator 9, and the LED touch screen 8 may be integrated together or implemented independently. In the implementation process, each operation of the above method or each module may be completed by a hardware integrated logic circuit in the processor 5 or instructions in a form of software.

Optionally, the LED touch screen 8 is electrically connected with a power supply unit. And the power supply unit supplies electric power to the processor 5, the communicator 9, and the LED touch screen 8. The power supply unit includes dry batteries or rechargeable batteries.

In an embodiment, in order to increase the dispersion of tensile forces on the user's teeth and the user's comfort, materials of the upper bite pad 2 and the lower bite pad 3 include one or a combination of foam, polylactic acid, nylon plastic, photosensitive resin, silicone, rubber, latex, acrylonitrile butadiene styrene (ABS) plastic, polyvinyl chloride (PVC) plastics, silicone resins, and acrylic-based resins.

As described above, the present disclosure provides an intelligent mouth opening rehabilitation device. The intelligent mouth opening rehabilitation device includes: a body, an end of the body being provided with an upper bite pad and a lower bite pad; a pressure sensor, provided in the upper bite pad and/or the lower bite pad, for detecting pressure data on the upper bite pad and/or the lower bite pad; a stepping motor, for driving the upper bite pad and/or the lower bite pad to swing longitudinally; and a processor, for controlling the stepping motor to operate or stop according to the pressure data and/or a preset training instruction, thereby driving the upper bite pad and/or the lower bite pad to swing longitudinally or to stop.

Compared with traditional mouth opening rehabilitation devices that require manual measurement of the opening angle or opening distance per use and require the need to manually control the training duration, the intelligent mouth opening rehabilitation device in the present disclosure controls automatically the upper bite pad and/or the lower bite pad to swing longitudinally, so that the mouth opening training is more accurate, more scientific and more controllable. Therefore, the rehabilitation effect can be greatly improved by using the intelligent mouth opening rehabilitation device in the present disclosure.

As described above, the present disclosure effectively overcomes various defects in the prior art and has a high industrial value.

The foregoing embodiments only describe several implementations of the present disclosure, and their description is specific and detailed, but cannot therefore be understood as a limitation to the patent scope of the present disclosure. Therefore, the protection scope of the present disclosure shall be subject to the appended claims.

## Claims

1. An intelligent mouth opening rehabilitation device, comprising:
a body (1), wherein an end of the body (1) is provided with an upper bite pad (2) and a lower bite pad (3);
a pressure sensor (6), provided in the upper bite pad (2) and/or the lower bite pad (3), for detecting pressure data on the upper bite pad (2) and/or the lower bite pad (3);
a stepping motor (4), for driving the upper bite pad (2) and/or the lower bite pad (3) to swing longitudinally; and
a processor (5), for controlling the stepping motor (4) to operate or stop according to the pressure data and/or a preset training instruction, thereby driving the upper bite pad (2) and/or the lower bite pad (3) to swing longitudinally or to stop;
wherein the processor (5) for
controlling the stepping motor (4) to operate or stop according to the pressure data is configured to compare the pressure data with a preset pressure threshold in real time; if the pressure data are lower than the preset pressure threshold, the processor (5) controls the stepping motor (4) to operate forward, to enable the upper bite pad (2) to swing up a distance and/or the lower bite pad (3) to swing down a distance, then returns to the first operation; and if the pressure data reach the preset pressure threshold, the processor (5) controls the stepping motor (4) to stop operating, to enable the upper bite pad (2) and/or the lower bite pad (3) to stop swinging; wherein the preset pressure threshold is set by testing a patient's pain performance in combination with a physician's observations when the physician instructs the patient on the use of the mouth opening rehabilitation device in an early stage;
wherein the processor (5) for
controlling the stepping motor (4) to operate or stop according to the pressure data further includes an operation wherein the pressure sensor (6) detects the pressure data in real time to determine whether the pressure data reach a second pressure threshold; wherein the second pressure threshold is greater than the preset pressure, so that the patient increases a pressure exerted on the upper bite pad (2) and the lower bite pad (3) through upper and lower jaws of a mouth of the patient to reduce an opening angle between the upper bite pad (2) and the lower bite pad (3), when the patient wants to interrupt a mouth opening training or when the patient wants to reduce the opening angle due to increasing soreness of the oral-jaw muscles; wherein if the pressure data reach the second pressure threshold, the processor (5) controls the stepping motor (4) to operate reversely, thereby enabling the upper bite pad (2) to swing down a distance and/or the lower bite pad (3) to swing up a distance, then returns to the first operation.

2. The intelligent mouth opening rehabilitation device according to claim 1, wherein the preset training instruction comprises: a preset training duration, and a preset opening angle or a preset opening distance between the upper bite pad (2) and the lower bite pad (3).

3. The intelligent mouth opening rehabilitation device according to claim 2, wherein an angle sensor (7) is provided in the upper bite pad (2) and/or the lower bite pad (3) and used to collect and characterize the opening angle between the upper bite pad (2) and the lower bite pad (3); or an infrared rangefinder (7) is provided in the upper bite pad (2) and/or the lower bite pad (3) and used to collect and characterize an opening distance between the upper bite pad (2) and the lower bite pad (3).

4. The intelligent mouth opening rehabilitation device according to claim 3, wherein the processor (5) controlling the stepping motor (4) to operate or stop according to the preset opening angle or the preset opening distance in the preset training instruction comprises:
the processor (5) controls the stepping motor (4) to operate forward according to the preset opening angle or the preset opening distance, to enable the upper bite pad (2) to swing up a distance and/or the lower bite pad (3) to swing down a distance; and
when the angle sensor (7) detects that the opening angle between the upper bite pad (2) and the lower bite pad (3) reaches the preset opening angle, or when the infrared rangefinder (7) detects that the opening distance between the upper bite pad (2) and the lower bite pad (3) reaches the preset opening distance, the processor (5) controls the stepping motor (4) to stop operating.

5. The intelligent mouth opening rehabilitation device according to claim 2, wherein the processor (5) controlling the stepping motor (4) to operate or stop according to the preset training duration in the preset training instruction, comprises:
when the stepping motor (4) starts and enters a stop state, a countdown is carried out based on the preset training duration; and
when a timer detects that the training duration has been reached, a speaker emits a prompt tone, and the processor (5) controls the stepping motor (4) to operate reversely, so that the upper bite pad (2) swings down to its starting position and/or the lower bite pad (3) swings up to its starting position.

6. The intelligent mouth opening rehabilitation device according to claim 2, wherein the intelligent mouth opening rehabilitation device further comprises:
a light emitting diode touch screen (8), located on one side of the body (1), used to display real-time pressure data, a training duration, training times, and an opening angle or an opening distance, and/or, used to manually set preset training times and the preset opening angle or the preset opening distance; and
a communicator (9), communicating with an external device, used to send the real-time pressure data, the training duration, the training times, and the opening angle or the opening distance to the external device, and/or, used to receive the preset training times and the preset opening angle or the preset opening distance from the external device.

7. The intelligent mouth opening rehabilitation device according to claim 6, wherein a communication mode of the communicator (9) comprises a wired communication mode and/or a wireless communication mode:
wherein the wired communication mode comprises one or a combination of USB1.0/2.0/3.x, Micro USB, Mini USB, serial interface and parallel interface;
wherein the wireless communication mode comprises one or a combination of 2G/3G/4G/5G, Bluetooth, Infrared, NB-IoT, Rola, Zigbee, MavLink, WIFI, NFC, GPRS, GSM and Ethernet.

8. The intelligent mouth opening rehabilitation device according to claim 1, wherein the upper bite pad (2) and the lower bite pad (3) are both provided with a corresponding stepping motor (4) respectively; or, one of the upper bite pad (2) and the lower bite pad (3) is provided with a corresponding stepping motor (4) and the other of the upper bite pad (2) and the lower bite pad (3) is fixed.

## Patentansprüche

1. Intelligente Vorrichtung zur Rehabilitation der Mundöffnung, umfassend:
einen Körper (1), wobei ein Ende des Körpers (1) mit einer oberen Beißauflage (2) und einer unteren Beißauflage (3) versehen ist;
einen Drucksensor (6), der in der oberen Beißauflage (2) und/oder der unteren Beißauflage (3) vorgesehen ist, um Druckdaten auf der oberen Beißauflage (2) und/oder der unteren Beißauflage (3) zu erfassen;
einen Schrittmotor (4), um die obere Beißauflage (2) und/oder die untere Beißauflage (3) anzutreiben, um in Längsrichtung zu schwenken; und
einen Prozessor (5), um den Schrittmotor (4) zu steuern, um gemäß den Druckdaten und/oder einer voreingestellten Trainingsanweisung zu betätigen oder anzuhalten, um dadurch die obere Beißauflage (2) und/oder die untere Beißauflage (3) anzutreiben, um in Längsrichtung zu schwenken oder anzuhalten;
wobei der Prozessor (5) zum Steuern des Schrittmotors (4), um diesen gemäß den Druckdaten zu betätigen oder anzuhalten, konfiguriert ist, um die Druckdaten mit einem voreingestellten Druckschwellenwert in Echtzeit zu vergleichen; wenn die Druckdaten niedriger als der voreingestellte Druckschwellenwert sind, steuert der Prozessor (5) den Schrittmotor (4), um vorwärts zu arbeiten, um zu ermöglichen, dass die obere Beißauflage (2) um eine Strecke nach oben schwenkt und/oder die untere Beißauflage (3) um eine Strecke nach unten schwenkt, und kehrt dann zu der ersten Operation zurück; und wenn die Druckdaten den voreingestellten Druckschwellenwert erreichen, steuert der Prozessor (5) den Schrittmotor (4), um den Betrieb anzuhalten, um zu ermöglichen, dass die obere Beißauflage (2) und/oder die untere Beißauflage (3) aufhören zu schwenken; wobei der voreingestellte Druckschwellenwert durch Testen der Schmerzempfindung eines Patienten in Kombination mit Beobachtungen eines Arztes eingestellt wird, wenn der Arzt den Patienten über die Verwendung der Vorrichtung zur Rehabilitation der Mundöffnung in einem frühen Stadium anweist;
wobei der Prozessor (5) zum Steuern des Schrittmotors (4), um diesen gemäß den Druckdaten zu betätigen oder anzuhalten, ferner eine Operation beinhaltet, wobei der Drucksensor (6) die Druckdaten in Echtzeit erfasst, um zu bestimmen, ob die Druckdaten einen zweiten Druckschwellenwert erreichen; wobei der zweite Druckschwellenwert größer als der voreingestellte Druck ist, so dass der Patient einen Druck erhöht, der auf die obere Beißauflage (2) und die untere Beißauflage (3) durch Ober- und Unterkiefer eines Mundes des Patienten ausgeübt wird, um einen Öffnungswinkel zwischen der oberen Beißauflage (2) und der unteren Beißauflage (3) zu verringern, wenn der Patient ein Mundöffnungstraining unterbrechen möchte oder wenn der Patient den Öffnungswinkel aufgrund der zunehmenden Schmerzen der Mundkiefermuskulatur verringern möchte; wobei, wenn die Druckdaten den zweiten Druckschwellenwert erreichen, der Prozessor (5) den Schrittmotor (4) steuert, um umgekehrt zu arbeiten, um dadurch zu ermöglichen, dass die obere Beißauflage (2) um eine Strecke nach unten schwenkt und/oder die untere Beißauflage (3) um eine Strecke nach oben schwenkt, und dann zu der ersten Operation zurückkehrt.

2. Intelligente Vorrichtung zur Rehabilitation der Mundöffnung nach Anspruch 1, wobei die voreingestellte Trainingsanweisung umfasst: eine voreingestellte Trainingsdauer und einen voreingestellten Öffnungswinkel oder einen voreingestellten Öffnungsabstand zwischen der oberen Beißauflage (2) und der unteren Beißauflage (3).

3. Intelligente Vorrichtung zur Rehabilitation der Mundöffnung nach Anspruch 2, wobei ein Winkelsensor (7) in der oberen Beißauflage (2) und/oder der unteren Beißauflage (3) vorgesehen ist und verwendet wird, um den Öffnungswinkel zwischen der oberen Beißauflage (2) und der unteren Beißauflage (3) zu erfassen und zu charakterisieren; oder ein Infrarotentfernungsmesser (7) in der oberen Beißauflage (2) und/oder der unteren Beißauflage (3) vorgesehen ist und verwendet wird, um einen Öffnungsabstand zwischen der oberen Beißauflage (2) und der unteren Beißauflage (3) zu erfassen und zu charakterisieren.

4. Intelligente Vorrichtung zur Rehabilitation der Mundöffnung nach Anspruch 3, wobei der Prozessor (5), der den Schrittmotor (4) steuert, um diesen gemäß dem voreingestellten Öffnungswinkel oder dem voreingestellten Öffnungsabstand in der voreingestellten Trainingsanweisung zu betätigen oder anzuhalten, umfasst:
der Prozessor (5) steuert den Schrittmotor (4), um gemäß dem voreingestellten Öffnungswinkel oder dem voreingestellten Öffnungsabstand vorwärts zu betätigen, um zu ermöglichen, dass die obere Beißauflage (2) um eine Strecke nach oben schwenkt und/oder die untere Beißauflage (3) um eine Strecke nach unten schwenkt; und
wenn der Winkelsensor (7) erfasst, dass der Öffnungswinkel zwischen der oberen Beißauflage (2) und der unteren Beißauflage (3) den voreingestellten Öffnungswinkel erreicht, oder wenn der Infrarotentfernungsmesser (7) erfasst, dass der Öffnungsabstand zwischen der oberen Beißauflage (2) und der unteren Beißauflage (3) den voreingestellten Öffnungsabstand erreicht, der Prozessor (5) den Schrittmotor (4) steuert, um den Betrieb anzuhalten.

5. Intelligente Vorrichtung zur Rehabilitation der Mundöffnung nach Anspruch 2, wobei der Prozessor (5), der den Schrittmotor (4) steuert, um diesen gemäß der voreingestellten Trainingsdauer in der voreingestellten Trainingsanweisung zu betätigen oder anzuhalten, umfasst:
wenn der Schrittmotor (4) startet und in einen Anhaltezustand eintritt, ein Countdown basierend auf der voreingestellten Trainingsdauer ausgeführt wird; und
wenn ein Timer erfasst, dass die Trainingsdauer erreicht wurde, ein Lautsprecher einen Aufforderungston ausgibt, und der Prozessor (5) den Schrittmotor (4) steuert, um umgekehrt zu arbeiten, so dass die obere Beißauflage (2) nach unten zu ihrer Ausgangsposition schwenkt und/oder die untere Beißauflage (3) nach oben zu ihrer Ausgangsposition schwenkt.

6. Intelligente Vorrichtung zur Rehabilitation der Mundöffnung nach Anspruch 2, wobei die intelligente Vorrichtung zur Rehabilitation der Mundöffnung ferner umfasst:
einen Leuchtdioden-Berührungsbildschirm (8), der sich auf einer Seite des Körpers (1) befindet und verwendet wird, um Echtzeit-Druckdaten, eine Trainingsdauer, Trainingszeiten und einen Öffnungswinkel oder einen Öffnungsabstand anzuzeigen, und/oder verwendet wird, um voreingestellte Trainingszeiten und den voreingestellten Öffnungswinkel oder den voreingestellten Öffnungsabstand manuell einzustellen; und
einen Kommunikator (9), der mit einer externen Vorrichtung kommuniziert und verwendet wird, um die Echtzeit-Druckdaten, die Trainingsdauer, die Trainingszeiten und den Öffnungswinkel oder den Öffnungsabstand an die externe Vorrichtung zu senden, und/oder verwendet wird, um die voreingestellten Trainingszeiten und den voreingestellten Öffnungswinkel oder den voreingestellten Öffnungsabstand von der externen Vorrichtung zu empfangen.

7. Intelligente Vorrichtung zur Rehabilitation der Mundöffnung nach Anspruch 6, wobei ein Kommunikationsmodus des Kommunikators (9) einen drahtgebundenen Kommunikationsmodus und/oder einen drahtlosen Kommunikationsmodus umfasst:
wobei der drahtgebundene Kommunikationsmodus eine oder eine Kombination von USB1.0/2.0/3.x, Micro USB, Mini USB, serieller Schnittstelle und paralleler Schnittstelle umfasst;
wobei der drahtlose Kommunikationsmodus ein oder eine Kombination von 2G/3G/4G/5G, Bluetooth, Infrarot, NB-IoT, Rola, Zigbee, MavLink, WIFI, NFC, GPRS, GSM und Ethernet umfasst.

8. Intelligente Vorrichtung zur Rehabilitation der Mundöffnung nach Anspruch 1, wobei die obere Beißauflage (2) und die untere Beißauflage (3) beide jeweils mit einem entsprechenden Schrittmotor (4) versehen sind; oder dass eine von der oberen Beißauflage (2) und der unteren Beißauflage (3) mit einem entsprechenden Schrittmotor (4) versehen ist und die andere von der oberen Beißauflage (2) und der unteren Beißauflage (3) fixiert ist.

## Revendications

1. Dispositif de rééducation d'ouverture de bouche intelligent, comprenant :
un corps (1), dans lequel une extrémité du corps (1) est pourvue d'un tampon de morsure supérieur (2) et d'un tampon de morsure inférieur (3) ;
un capteur de pression (6), prévu dans le tampon de morsure supérieur (2) et/ou le tampon de morsure inférieur (3), pour détecter des données de pression sur le tampon de morsure supérieur (2) et/ou le tampon de morsure inférieur (3)
un moteur pas à pas (4), pour entraîner le tampon de morsure supérieur (2) et/ou le tampon de morsure inférieur (3) à osciller longitudinalement ; et
un processeur (5), pour commander le moteur pas à pas (4) pour qu'il fonctionne ou s'arrête en fonction des données de pression et/ou d'une instruction d'entraînement prédéfinie, entraînant ainsi le tampon de morsure supérieur (2) et/ou le tampon de morsure inférieur (3) à osciller longitudinalement ou à s'arrêter
dans lequel le processeur (5) pour commander le moteur pas à pas (4) pour qu'il fonctionne ou s'arrête en fonction des données de pression est configuré pour comparer les données de pression à un seuil de pression prédéfini en temps réel ; si les données de pression sont inférieures au seuil de pression prédéfini, le processeur (5) commande le moteur pas à pas (4) pour qu'il fonctionne vers l'avant, pour permettre au tampon de morsure supérieur (2) d'osciller vers le haut d'une distance et/ou au tampon de morsure inférieur (3) d'osciller vers le bas d'une distance, puis retourne à la première opération ; et si les données de pression atteignent le seuil de pression prédéfini, le processeur (5) commande le moteur pas à pas (4) pour qu'il arrête de fonctionner, pour permettre au tampon de morsure supérieur (2) et/ou au tampon de morsure inférieur (3) d'arrêter d'osciller ; dans lequel le seuil de pression prédéfini est établi en testant la performance de la douleur d'un patient en combinaison avec des observations d'un médecin lorsque le médecin demande au patient l'utilisation du dispositif de rééducation d'ouverture de bouche à un stade précoce ;
dans lequel le processeur (5) pour commander le moteur pas à pas (4) pour qu'il fonctionne ou s'arrête en fonction des données de pression inclut en outre une opération dans laquelle le capteur de pression (6) détecte les données de pression en temps réel pour déterminer si les données de pression atteignent un second seuil de pression ; dans lequel le second seuil de pression est supérieur à la pression prédéfinie, de sorte que le patient augmente une pression exercée sur le tampon de morsure supérieur (2) et le tampon de morsure inférieur (3) à travers les mâchoires supérieure et inférieure d'une bouche du patient pour réduire un angle d'ouverture entre le tampon de morsure supérieur (2) et le tampon de morsure inférieur (3), lorsque le patient veut interrompre un entraînement d'ouverture de bouche ou lorsque le patient veut réduire l'angle d'ouverture en raison de la douleur croissante des muscles de mâchoire buccale; dans lequel si les données de pression atteignent le second seuil de pression, le processeur (5) commande le moteur pas à pas (4) pour qu'il fonctionne en sens inverse, permettant ainsi au tampon de morsure supérieur (2) d'osciller vers le bas d'une distance et/ou au tampon de morsure inférieur (3) d'osciller vers le haut d'une distance, puis retourne à la première opération.

2. Dispositif de rééducation d'ouverture de bouche intelligent selon la revendication 1, dans lequel l'instruction d'entraînement prédéfinie comprend : une durée d'entraînement prédéfinie, et un angle d'ouverture prédéfini ou une distance d'ouverture prédéfinie entre le tampon de morsure supérieur (2) et le tampon de morsure inférieur (3).

3. Dispositif de rééducation d'ouverture de bouche intelligent selon la revendication 2, dans lequel un capteur d'angle (7) est prévu dans le tampon de morsure supérieur (2) et/ou le tampon de morsure inférieur (3) et utilisé pour collecter et caractériser l'angle d'ouverture entre le tampon de morsure supérieur (2) et le tampon de morsure inférieur (3) ; ou un télémètre infrarouge (7) est prévu dans le tampon de morsure supérieur (2) et/ou le tampon de morsure inférieur (3) et utilisé pour collecter et caractériser une distance d'ouverture entre le tampon de morsure supérieur (2) et le tampon de morsure inférieur (3).

4. Dispositif de rééducation d'ouverture de bouche intelligent selon la revendication 3, dans lequel le processeur (5) commandant le moteur pas à pas (4) pour qu'il fonctionne ou s'arrête en fonction de l'angle d'ouverture prédéfini ou de la distance d'ouverture prédéfinie dans l'instruction d'entraînement prédéfinie comprend :
le processeur (5) commande le moteur pas à pas (4) pour qu'il fonctionne vers l'avant en fonction de l'angle d'ouverture prédéfini ou de la distance d'ouverture prédéfinie, pour permettre au tampon de morsure supérieur (2) d'osciller vers le haut d'une distance et/ou au tampon de morsure inférieur (3) d'osciller vers le bas d'une distance ; et
lorsque le capteur d'angle (7) détecte que l'angle d'ouverture entre le tampon de morsure supérieur (2) et le tampon de morsure inférieur (3) atteint l'angle d'ouverture prédéfini, ou lorsque le télémètre infrarouge (7) détecte que la distance d'ouverture entre le tampon de morsure supérieur (2) et le tampon de morsure inférieur (3) atteint la distance d'ouverture prédéfinie, le processeur (5) commande le moteur pas à pas (4) pour qu'il arrête de fonctionner.

5. Dispositif de rééducation d'ouverture de bouche intelligent selon la revendication 2, dans lequel le processeur (5) commandant le moteur pas à pas (4) pour qu'il fonctionne ou s'arrête en fonction de la durée d'entraînement prédéfinie dans l'instruction d'entraînement prédéfinie, comprend :
lorsque le moteur pas à pas (4) démarre et entre dans un état d'arrêt, un compte à rebours est effectué sur la base de la durée d'entraînement prédéfinie ; et
lorsqu'un horloge en temps réel détecte que la durée d'entraînement a été atteinte, un haut-parleur émet une tonalité d'invite, et le processeur (5) commande le moteur pas à pas (4) pour qu'il fonctionne en sens inverse, de sorte que le tampon de morsure supérieur (2) oscille vers le bas jusqu'à sa position de démarrage et/ou le tampon de morsure inférieur (3) oscille vers le haut jusqu'à sa position de démarrage.

6. Dispositif de rééducation d'ouverture de bouche intelligent selon la revendication 2, dans lequel le dispositif de rééducation d'ouverture de bouche intelligent comprend en outre :
un écran tactile à diode électroluminescente (8), situé sur un côté du corps (1), utilisé pour afficher des données de pression en temps réel, une durée d'entraînement, des temps d'entraînement, et un angle d'ouverture ou une distance d'ouverture, et/ou, utilisé pour établir manuellement des temps d'entraînement prédéfinis et l'angle d'ouverture prédéfini ou la distance d'ouverture prédéfinie ; et
un communicateur (9), communiquant avec un dispositif externe, utilisé pour envoyer les données de pression en temps réel, la durée d'entraînement, les temps d'entraînement, et l'angle d'ouverture ou la distance d'ouverture au dispositif externe, et/ou, utilisé pour recevoir les temps d'entraînement prédéfinis et l'angle d'ouverture prédéfini ou la distance d'ouverture prédéfinie à partir du dispositif externe.

7. Dispositif de rééducation d'ouverture de bouche intelligent selon la revendication 6, dans lequel un mode de communication du communicateur (9) comprend un mode de communication filaire et/ou un mode de communication sans fil :
dans lequel le mode de communication filaire comprend une ou une combinaison d'une interface série et d'une interface parallèle USB1.0/2.0/3.x, Micro USB, Mini USB ;
dans lequel le mode de communication sans fil comprend une ou une combinaison de 2G/3G/4G/5G, Bluetooth, infrarouge, NB-IoT, Rola, Zigbee, MavLink, WIFI, NFC, GPRS, GSM et Ethernet.

8. Dispositif de rééducation d'ouverture de bouche intelligent selon la revendication 1, dans lequel le tampon de morsure supérieur (2) et le tampon de morsure inférieur (3) sont tous deux pourvus d'un moteur pas à pas correspondant (4) respectivement ; ou, que l'un du tampon de morsure supérieur (2) et du tampon de morsure inférieur (3) est pourvu d'un moteur pas à pas correspondant (4) et l'autre du tampon de morsure supérieur (2) et du tampon de morsure inférieur (3) est fixe.
